# EUROPÄISCHE PATENTSCHRIFT

(11) **EP 0 945 449 B1**
(45) Veröffentlichungstag und Bekanntmachung des Hinweises auf die Patenterteilung: **23.05.2001**
(21) Anmeldenummer: 99105589.8
(22) Anmeldetag: 18.03.1999
(51) Int. Cl.: C07D 275/06, C07C 311/16

(54) **Verfahren zur Herstellung von Saccharincarbonsäurehalogeniden**
Process for the preparation of saccharincarboxylic acid halides
Procédé de préparation d'halogénures d'acides saccharinecarboxyliques

(30) Priorität: 25.03.1998 DE 19813014
(43) Veröffentlichungstag der Anmeldung: 29.09.1999
(73) Patentinhaber: BASF AKTIENGESELLSCHAFT, 67056 Ludwigshafen (DE)
(72) Erfinder: Plath, Peter Dr., 67227 Frankenthal (DE); von Deyn, Wolfgang Dr., 67435 Neustadt (DE); Rack, Michael Dr., 69123 Heidelberg (DE); Misslitz, Ulf Dr., 67433 Neustadt (DE)

(56) Entgegenhaltungen:
- DE-A- 4 427 996

## Beschreibung

Die vorliegende Erfindung betrifft ein Verfahren zur Herstellung von Saccharincarbonsäurehalogeniden der allgemeinen Formel 1, 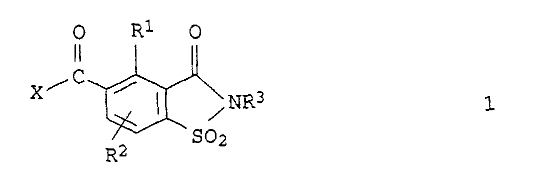 in der die Substituenten die folgende Bedeutung haben:
- X: Halogen,
- R¹, R²: Wasserstoff, C₁-C₆-Alkyl, C₃-C₈-Cycloalkyl, C₁-C₄-Alkoxy, ggf. durch C₁-C₄-Alkyl, Fluor oder C₁-C₄-Alkoxy substituiertes Benzyl oder Phenyl,
- R³: Wasserstoff, C₁-C₆-Alkyl, C₃-C₈-Cycloalkyl sowie ggf. durch C₁-C₄-Alkyl, Halogen, C₁-C₄-Halogenalkyl oder C₁-C₄-Alkoxy ein- oder mehrfach substituiertes Benzyl oder Phenyl.

Ferner betrifft die vorliegende Erfindung Verfahren zur Herstellung der Zwischenprodukte, aus denen die Saccharincarbonsäurehalogenide 1 hergestellt werden können.

Die Saccharincarbonsäurehalogenide 1 sind wichtige Zwischenprodukte bei der Herstellung herbizider Wirkstoffe, wie sie beispielsweise in der WO 96/05182, WO 96/05197, WO 96/95198 und P 19709697.2 beschrieben sind.

In diesen Anmeldungen sind herbizide Saccharin-Derivate beschrieben, die über eine Carbonylgruppe am Aromaten mit einem Cyclohexan-1,3-dionring, einem Hydroxypyrazolring, einem Isoxazolring oder einem Cyclohexan-1,3,5-trionring verbunden sind.

Verfahren zur Herstellung von Saccharin-Derivaten sind bekannt. So wird in der DE 36 07 343 ein Verfahren zur Herstellung von 4-Hydroxy-1,2-benzisothiazol-3(2H)-on-1,1-dioxid beschrieben, bei dem substituierte 1,2-Benzisothiazol-1,1-dioxide mit Alkoholaten nucleophil substituiert werden. Als Zwischenprodukt wurde auch 4-Chlorsaccharin-5-carbonsäure beschrieben, die durch Oxidation von 3-Chlor-2,4-dimethylbenzolsulfonamid mit Kaliumpermanganat hergestellt wurde, d.h. die beiden Carboxylfunktionen wurden durch Oxidation zweier Methylgruppen eingeführt bei gleichzeitig oxidativem Ringschluß. Nachteilig ist bei diesem Verfahren, daß bei Vorhandensein mehrerer funktioneller Gruppen keine selektive Oxidation möglich ist.

In der WO 96/05184 wird ein Verfahren zur Herstellung von Saccharincarbonsäuren und -estern beschrieben durch Umsetzung von brom- oder iodsubstituierten Saccharinderivaten bzw. deren offenkettigen Carbonamid-Sulfonamid-Derivaten mit Kohlenmonoxid und Wasser bzw. einem Alkohol in Gegenwart einer Base und eines Übergangsmetallkatalysators.

Dieses Verfahren hat den Nachteil, daß bei Verwendung von Aryljodiden unerwünschte Nebenprodukte entstehen.

Aufgabe der vorliegenden ERfindung war es, ein neues Verfahren zur Herstellung von Saccharincarbonsäurehalogeniden der allgemeinen Formel 1 zur Verfügung zu stellen, das von einfach zugänglichen Ausgangskomponenten ausgeht und nicht die Nachteile der Verfahren des Standes der Technik aufweist.

Weiterhin wurde ein Verfahren zur Herstellung des Isophthalsäuresulfonamids 2 gefunden durch Verseifung eines 5-Cyanosaccharins 3 mit wäßriger Alkalilauge 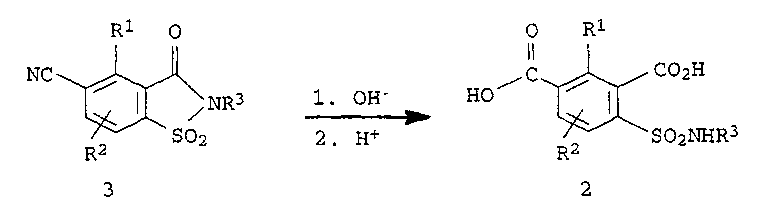 und anschließender Zugabe einer Säure.

Darüber hinaus wurde noch ein Verfahren zur Herstellung des 5-Cyanosaccharin 3 gefunden durch Umsetzung eines Dicyanobenzolsulfonylchlorids 4 mit einem Amin der Formel R³-NH₂ zum Iminosaccharinderivat 5 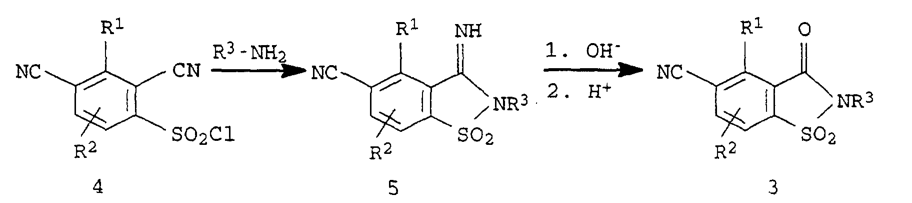 und anschließender saurer Hydrolyse.

Es wurde ein Verfahren zur Herstellung von Saccharincarbonsäurehalogeniden der allgemeinen Formel 1 gefunden, 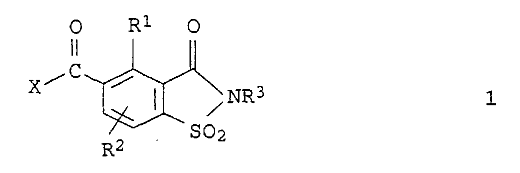 in der die Substituenten die folgende Bedeutung haben:
- X: Halogen,
- R¹, R²: Wasserstoff, C₁-C₆-Alkyl, C₃-C₈-Cycloalkyl, C₁-C₄-Alkoxy, ggf. durch C₁-C₄-Alkyl, Fluor oder C₁-C₄-Alkoxy substituiertes Benzyl oder Phenyl,
- R³: Wasserstoff, C₁-C₆-Alkyl, C₃-C₈-Cycloalkyl sowie ggf. durch C₁-C₄-Alkyl, Halogen, C₁-C₄-Halogenalkyl oder C₁-C₄-Alkoxy ein- oder mehrfach substituiertes Benzyl oder Phenyl,
indem man ein Isophthalsäuresulfonamid 2 mit einem anorganischen Säurehalogenid zum Saccharincarbonsäurehalogenid 1 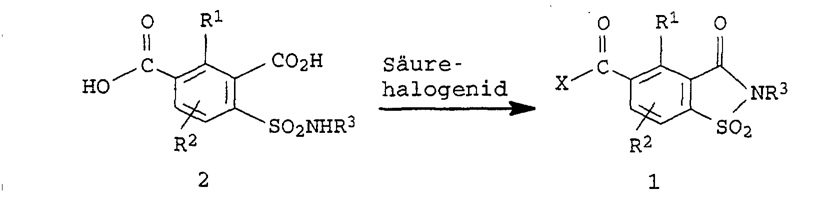 umsetzt.

Die für die Substituenten X und R¹ - R³ oder als Reste an Phenyl- und Benzylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: Methyl, Ethyl, n-Propyl, 1-Methylethyl, Butyl, 1-Methylpropyl, 2-Methylpropyl und 1,1-Dimethylethyl;
- C₁-C₆-Alkyl : C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, l-Ethyl-l-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₄-Alkoxy : Methoxy, Ethoxy, Propoxy, 1-Methylethoxy, Butoxy, 1-Methylpropoxy, 2-Methylpropoxy und 1,1-Dimethylethoxy;
- C₃-C₈-Cycloalkyl: Cyclopropyl, Cyclobutyl, Cyclopentyl, Cyclohexyl, Cycloheptyl und Cyclooctyl.

Bevorzugt werden nach dem erfindungsgemäßen Verfahren Saccharincarbonsäurehalogenide der allgemeinen Formel 1 hergestellt, in der die Substituenten die folgende Bedeutung haben:
- X: Chlor,
- R¹, R²: Wasserstoff, C₁-C₆-Alkyl, C₁-C₄-Alkoxy oder ggf. durch C₁-C₄-Alkyl, Fluor oder C₁-C₄-Alkoxy substituiertes Benzyl,
- R³: Wasserstoff, C₁-C₆-Alkyl, C₃-C₈-Cycloalkyl, Phenyl oder Benzyl.

Weiterhin bevorzugt werden Verbindungen 1 mit den Substituenten
- X: Chlor,
- R¹, R²: Wasserstoff, Methyl, Ethyl oder Methoxy,
- R³: Wasserstoff, Methyl, Ethyl, Isopropyl, Cyclohexyl, Benzyl oder Phenyl
hergestellt.

Besonders bevorzugt werden Verbindungen 1 mit den Substituenten
- X: Chlor,
- R¹: Methyl, Ethyl oder Methoxy,
- R²: Wasserstoff,
- R³: Wasserstoff, Methyl, Ethyl, Benzyl oder Phenyl
hergestellt.

Das gesamte Verfahren zur Herstellung der Saccharincarbonsäurehalogenide 1 erfolgt nach folgenden Stufen:

### Stufe 1

Die Synthesefolge beginnt mit der Herstellung des 3-Aminobenzonitrils 7 aus dem 3-Halogenanilin 6 und CuCN, 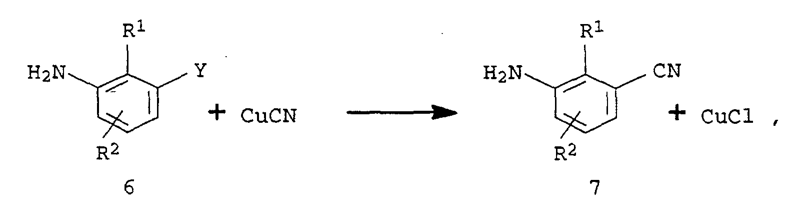 wobei Y für Cl, Br oder J steht, bevorzugt Cl. Geeignete Lösungsmittel sind organische Lösungsmittel wie beispielsweise N-Methylpyrrolidon, Pyridin, Dimethylformamid oder Tetramethylharnstoff.

Bevorzugt wird N-Methylpyrrolidon verwendet. Die Umsetzung erfolgt bei Temperaturen von 90 bis 240°C, bevorzugt 220 bis 230°C. Bei Verwendung von N-Methylpyrrolidon wird vorzugsweise beim Siedepunkt des Lösungsmittels gearbeitet. Die Reaktionszeit beträgt 6 bis 18 h, bevorzugt 8 bis 12 h.

Bei Arbeiten mit DMF oder Pyridin ist es von Vorteil, unter Eigendruck auf Temperaturen bis 240°C zu gehen.

### Stufe 2

Das Schwefelatom des späteren Saccharinrings wird durch Rhodanierung von 7 mit Dirhodan eingeführt:

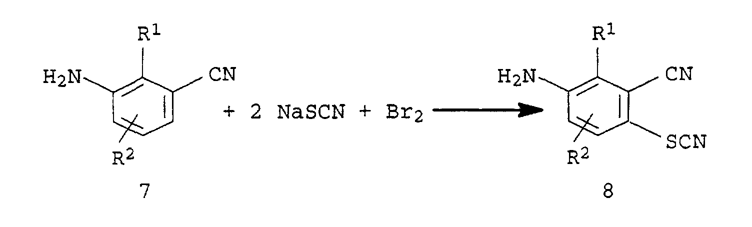

Die Umsetzung erfolgt in an sich bekannter Weise bei Temperaturen von -20°C bis +25°C, bevorzugt bei -5°C bis +5°C. Als Solvens eignen sich Alkohole wie Methanol oder Ethanol, aber auch Essigsäure, Propionsäure oder Isobuttersäure. Bevorzugtes Lösungsmittel ist Methanol, dem zur Stabilisierung etwas Kaliumbromid beigefügt ist.

### Stufe 3

Das so erhaltene 3-Cyano-4-thiocyanoanilin 8 wird dann in einer Sandmeyer-Reaktion zum 4-Thiocyano-isophthalsäuredinitril 9 umgewandelt:

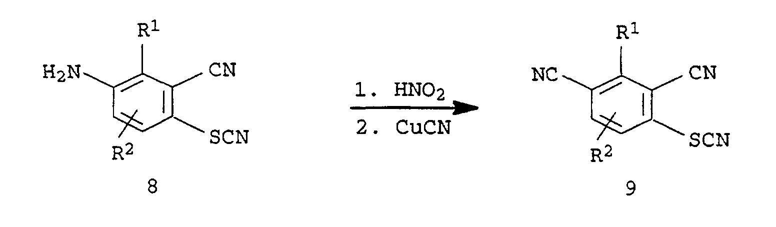

Geeignete Lösungsmittel sind organische Lösungsmittel wie beispielsweise Essigsäure oder Mischungen aus Essigsäure und konz. Salzsäure.

Bevorzugt wird das Gemisch Eisessig/konz. Salzsäure = 2,5:1 verwendet. Die Umsetzung erfolgt bei Temperaturen von -5 bis 25°C, bevorzugt -5 bis +5°C. Die Reaktionszeit beträgt 2 bis 16 h, bevorzugt 1 bis 3 h. Das Dinitril 9 fällt dabei als Komplex mit Cu(I) an und wird durch Behandlung mit O₂ in konz. Salzsäure freigesetzt.

### Stufe 4

Das Dinitril 9 wird anschließend mit wäßrigem Natriumsulfid zu 2,4-Dicyano-thiophenol 10 umgewandelt:

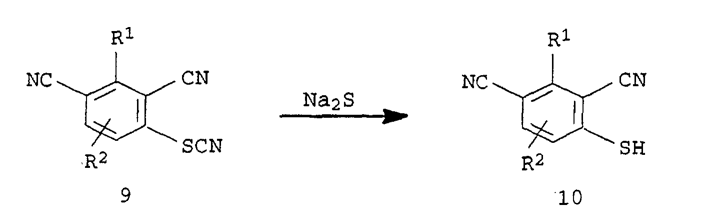

Geeignete Lösungsmittel sind organische Lösungsmittel wie beispielsweise Methanol oder Ethanol im Gemisch mit Wasser.

Bevorzugt wird wäßriges Methanol verwendet. Die Umsetzung erfolgt bei Temperaturen von 5 bis 30°C, bevorzugt 15 bis 25°C. Die Reaktionszeit beträgt 2 bis 8 h, bevorzugt 2 bis 3 h.

### Stufe 5

Chlorierung von 10 führt zu 2,4-Dicyanobenzolsulfonylchlorid 4:

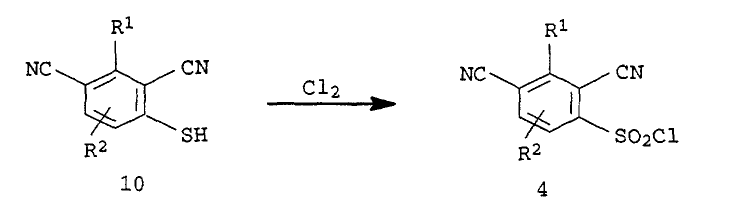

Geeignete Lösungsmittel sind organische Lösungsmittel wie beispielsweise Dichlormethan, 1,2-Dichlorethan, Chlorbenzol oder Essigsäure.

Bevorzugt wird ein Gemisch von Essigsäure und Dichlormethan verwendet. Die Umsetzung erfolgt bei Temperaturen von 0 bis 60°C, bevorzugt 25 bis 50°C. Die Reaktionszeit beträgt 2 bis 10 h, bevorzugt 4 bis 6 h, je nach der Einleitungsgeschwindigkeit des Chlors. Die Menge des einzuleitenden Chlors in mol ist mindestens fünfmal so viel, bezogen auf die Menge des verwendeten Thiophenols 10. Praktisch verwendet man jedoch einen Überschuß von Chlor, dadurch bestimmt, daß ein als Zwischenstufe auftretender Feststoff bei weiterer Chlorzugabe wieder in Lösung geht.

### Stufe 6

Einwirkung eines Amins der Formel R³-NH₂ liefert (statt des erwarteten Isophthalodinitrilsulfonamids) das Iminosaccharin-derivat 5, das durch saure Hydrolyse zu dem Cyanosaccharin 3 zerlegt wird:

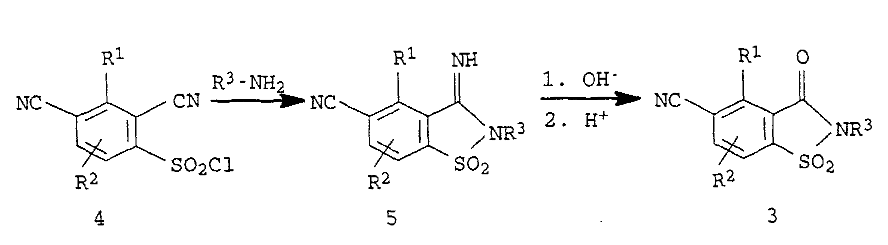

Geeignete Lösungsmittel sind organische Lösungsmittel wie beispielsweise Tetrahydrofuran, Dioxan oder Acetonitril.

Bevorzugt wird Tetrahydrofuran (THF) im Gemisch mit Wasser verwendet. Die Umsetzung erfolgt bei Temperaturen von 0 bis 40°C, bevorzugt 4 bis 25°C. Die Reaktionszeit beträgt 1 bis 16 h, bevorzugt 2 bis 8 h.

Als Amine werden Ammoniak, C₁-C₆-Alkylamine, C₃-C₈-Cycloalkylamine sowie ggf. durch C₁-C₄-Alkyl, Halogen, C₁-C₄-Halogenalkyl oder C₁-C₄-Alkoxy ein- oder mehrfach substituiertes Benzylamin oder Anilin verwendet.

Geeignete C₁-C₆-Alkylamine sind Methylamin, Ethylamin, Isopropylamin, n-Butylamin und Isobutylamin.

Bevorzugt sind Methylamin, Ethylamin und Isobutylamin.

Geeignete C₃-C₈-Cycloalkylamine sind Cyclopropylamin, Cyclobutylamin, Cyclopentylamin, Cyclohexylamin, Cycloheptylamin und Cyclooctylamin.

Bevorzugt sind Cyclopentylamin und Cyclohexylamin.

Geeignete substituierte Benzylamine oder Aniline sind 2-Chlorbenzylamin, Anilin und 2-Methylanilin, 3-Chloranilin, 3-CF₃-Anilin.

Bevorzugt sind Anilin und Benzylamin.

Ganz besonders bevorzugte Amine sind Ammoniak, Methylamin, Ethylamin, Isobutylamin und Anilin.

Die saure Hydrolyse wird mit organischen oder anorganischen Säuren durchgeführt. Bevorzugt wird Essigsäure verwendet.

### Stufe 7

Verseifung von 3 mit wäßriger Alkalilauge und anschließendem Ansäuern mit einer Säure liefert die Isophthalsäure 2:

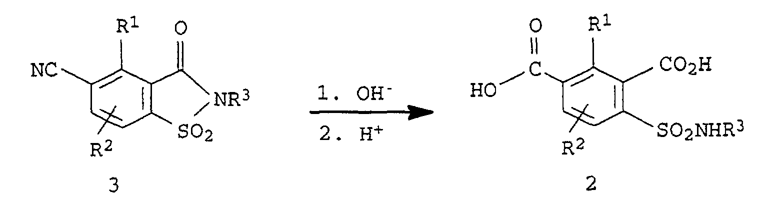

Die Verseifung von 3 erfolgt mit 20 bis 25 proz. NaOH oder KOH bei Temperaturen von 90 bis 100°C. Bevorzugt wird 20 proz. NaOH oder KOH verwendet. Die Dauer der Reaktion richtet sich nach dem Ende der Ammoniak-Entwicklung. Sie beträgt 6 bis 16 Std., bei Verwendung von 20 proz. KOH 6 bis 8 Std. Die Ansäuerung wird mit einer anorganischen oder organischen Säure durchgeführt. Bevorzugt wird konz. Salzsäure verwendet.

### Stufe 8

Erhitzen von 2 mit einem Säurehalogenid liefert das Saccharin-5-carbonsäurehalogenid 1

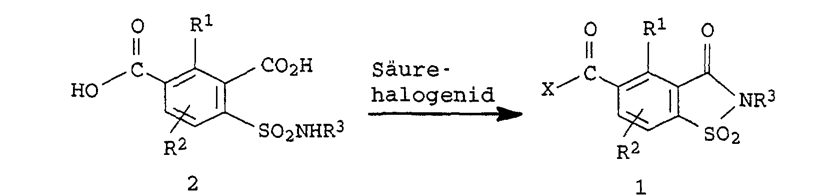

Als Lösungsmittel wird entweder überschüssiges Säurechlorid oder Toluol, Chlorbenzol, Xylole oder 1,2-Dichlorethan verwendet.

Als Säurehalogenide werden anorganische Säurechloride wie Thionylchlorid, Phosphoroxychlorid, Oxalylchlorid oder Phosgen verwendet.

Bevorzugt wird Thionylchlorid im Lösungsmittel Toluol verwendet. Die Temperatur beträgt 25 bis 130°C.

### Herstellungsbeispiele

### Stufe 1: 3-Amino-2-methylbenzonitril

283 g (2,0 mol) 3-Chlor-2-methylanilin werden in 250 ml N-Methylpyrrolidon gelöst und mit 200 g (2,23 mol) CuCN versetzt. Anschließend wird sechs Stunden unter N₂ bei 225°C gerührt. Nach Abkühlen auf 100°C wird das Reaktionsgemisch in 2,5 l 25 proz. Ammoniaklösung gegossen und eine Stunde bei 25°C gerührt. Durch den Luftsauerstoff wird dabei der Cu(I)-Nitrilkomplex des Produktes zu einem wasserlöslichen Cu(II)salz oxidiert, was an der Entstehung des intensiv blauen Tetraminkomplexes zu erkennen ist. Ein Teil des CuCl bleibt als weißer Feststoff ungelöst und ist leicht durch Absaugen zu entfernen. Das Filtrat wird mit ca. 2 l Methyl-tert.-butylether extrahiert, dann mit Ammoniakwasser und zuletzt dreimal mit Wasser gewaschen. Nach Trocknen der organischen Phase über Natriumsulfat und Einengen erhält man 255 g (89 % Rohausbeute) eines dunklen Öls, das durch Umkristallisation aus Cyclohexan/Toluol (5:1) zu reinigen ist.
Ausbeute nach Umkristallisation: 205 g (78 % d.Th.), Fp.: 93 bis 94°C

### Stufe 2: 2-Methyl-3-cyano-4-thiocyanoanilin

Man suspendiert 30 g NaBr in 1500 ml Methanol und fügt 212,8 g (1,52 mol) 3-Amino-2-methylbenzonitril sowie 246 g (3 mol) Natriumthiocyanat zu. Dann kühlt man auf -5°C und tropft langsam bei -5 bis 0°C 243 g (1,52 mol) Brom zu. Mit der Zeit fällt ein Niederschlag aus. Nach 16 Stunden Nachrühren bei 25°C ist die Umsetzung abgeschlossen.

### Aufarbeitung

Man gießt die Reaktionsmischung in 2,5 1 Wasser und saugt den ausgefallenen Feststoff (132 g) ab. Das Filtrat wird mit MTBE (Methyl-tert.Butylether) extrahiert und liefert nach Trocknen und Einengen des Extraktes weitere 144 g Rohprodukt. Beide Feststoffe werden vereinigt und aus Ethanol umkristallisiert.

Ausbeute nach Umkristallisation: 218 g (76 % d.Th.).

Das Produkt (Fp.: 143°C) ist nach IR und NMR rein.

Bei einem Wiederholungsversuch wurde eine Ausbeute von 80 % erzielt.

### Stufe 3: 2-Methyl-4-thiocyano-isophthalsäuredinitril

Man löst unter Erwärmen 189 g (1 mol) 2-Methyl-3-cyano-4-thiocyanoanilin in 1 kg Eisessig und fügt dann 400 g (4 mol) konz. HCl und nach 15 min Rühren 400 ml Wasser zu, so daß eine feinverteilte Suspension des Hydrochlorids entsteht. Nach kurzem Rühren (15 bis 30 min) wird bei -5 bis 0°C langsam die Lösung von 69 g (1 mol) Natriumnitrit in 140 ml Wasser zugetropft. In einem separaten Rührkolben löst man 245 g (5 mol) NaCN in einer Mischung aus 1,5 l Wasser und 136 g (2 mol) 25 proz. Ammoniakwasser und gibt 250 g (1 mol) CuSO₄·5 H₂O zu, worauf sich die dunkle Lösung eines Cu(I)cyanokomplexes bildet. Anschließend tropft man bei 25°C die zuvor bereitete und bei 0°C gehaltene Diazoniumlösung zügig in den Cu-Komplex ein, wobei man die Temperatur bis 40°C kommen läßt. Stickstoff wird frei. Nach Beendigung der Gasabspaltung wird noch 30 min nachrühren gelassen.

### Aufarbeitung

Das Produkt liegt als Feststoff (Nitrilkomplex mit Cu(I) vor). Eine Zerlegung dieses Komplexes durch Luftsauerstoff in ammoniakalischer Lösung verbietet sich, weil dabei der Thiocyanorest zum Disulfidrest umgewandelt wird.

Man saugt den ausgefallenen Feststoff ab und wäscht ihn dreimal mit Wasser. Das Filtrat enthält nicht-komplexiertes Produkt und wird deshalb mit 2 1 Methylenchlorid extrahiert. Der Feststoff wird dann in ein Rührgefäß gegeben und mit 1 l konz. HCl versetzt. Anschließend gießt man den Methylenchloridextrakt zu und läßt 15 min rühren. Nach Abtrennen der organischen Phase und Abfiltrieren ungelöst gebliebener Anteile wird die organische Unterphase dreimal mit Wasser gewaschen und nach Trocknung über Natriumsulfat eingeengt. Zur Abtrennung unerwünschter Bestandteile wird das Rohprodukt in Essigester gelöst, vom Ungelösten filtriert und die Lösung anschließend eingeengt. Ausbeute: 170 g (85 % d.Th.).

Das Produkt (Fp.: 93°C) ist nach IR und NMR >95 % rein und für weitere Umsetzungen ohne weitere Reinigung verwendbar.

### Stufe 4: 3-Methyl-2,4-dicyano-thiphenol

Man löst 170 g (0,85 mol) 2-Methyl-4-thiocyano-isophthalsäurenitril in 850 ml Methanol, tropft bei 25 bis 35°C eine Lösung von 110,5 g (mol) 60 proz. Natriumsulfid in 425 ml Wasser zu und läßt drei Stunden bei Raumtemperatur nachrühren. Danach wird mit 1000 ml Wasser versetzt und mit Methyl-tert.butylether (MTBE) extrahiert, um alle neutralen Bestandteile zu entfernen. Die wäßrige Phase wird durch Ansäuern mit konz. HCl auf pH 1 gebracht und das Thiophenol mit Methylenchlorid extrahiert. Nach dreimaligem Waschen des Extraktes mit Wasser wird die organische Phase abgetrennt, über Natriumsulfat getrocknet und eingeengt. Nach Trocknen des Rückstands im Ölpumpenvakuum erhält man 150 g (99 % d.Th.) eines orangefarbenen Feststoffs, der nach NMR rein ist.
Das Thiophenol ist bei Raumtemperatur nahezu geruchlos, was charakteristisch für Thiophenol mit elektronenanziehenden Substituenten ist. Aus dem gleichen Grund wird die Verbindung beim Stehen an der Luft nur sehr langsam zum Disulfid oxidiert.

### Stufe 5: 2,4-Dicyano-3-methyl-benzolsulfonylchlorid

Man löst 116 g (0,67 mol) 2,4-Dicyano-3-methyl-thiophenol in einer Mischung von 900 ml Essigsäure und 200 ml Methylenchlorid, fügt 48 g (2,67 mol) Wasser zu und leitet bei 25 bis 50°C innerhalb vier Stunden in kräftigem Strom Chlorgas in der Weise ein, daß am Blasenzähler kaum Abgas entweicht. Aus der Lösung fällt zwischendurch ein Feststoff aus, der sich im weiteren Verlauf der Chlorierung wieder löst. Nach vollständiger Auflösung wird noch ca. 15 min weiter Chlor eingeleitet, dann wird abkühlen gelassen, kurz mit Stickstoff gespült und dann am Rotationsverdampfer bei 50°C eingeengt. Der noch flüssige, auf 25°C abgekühlte Rückstand wird in Methylenchlorid aufgenommen, mit Eiswasser gewaschen, über Natriumsulfat getrocknet und eingeengt. Man isoliert 150 g (93 % d.Th.) eines Öls.
¹H NMR (CDCl₃, 270 MHz) δ [ppm] : 8,25 (d,1H), 8,15 (d,1H), 2,95 (s,3H)
IR (cm⁻¹): 2220, (CN), 1385, 1170 (beide für SO₂Cl)

### Stufe 6:

### 6.1 3-Imino-2,4-dimethyl-5-cyanosaccharin

Man legt eine Lösung aus 168 g (3,5 mol) 40 proz. wäßrigem Methylamin in 500 ml THF vor und tropft bei 25°C unter Eiskühlung eine Lösung von 150 g (0,62 mol) 2,4-Dicyano-3-methylsulfonsäurechlorid in 250 ml THF zu. Nach 6 Stunden Rühren bei Raumtemperatur wird am Rotationsverdampfer das THF und überschüssiges Methylamin entfernt. Anschließend wird der Rückstand zweimal mit 1 l verdünnter (1 proz.) HCl durchgerührt. Man isoliert einen orange gefärbten Feststoff, der ohne weitere Reinigung zur Hydrolyse eingesetzt wird.

### 6.2 2,4-Dimethyl-5-cyanosaccharin

Der oben erhaltene wasserfeuchte Feststoff (ca. 0,6 mol) wird in 1 1 Eisessig gelöst und drei Stunden zum Sieden erhitzt. Anschließend wird zur Trockene eingeengt, in 1 l Methylenchlorid aufgenommen und zur Entfernung von Ammoniumacetat dreimal mit Wasser ausgeschüttelt. Nach Trocknen der organischen Phase über Natriumsulfat und Abziehen des Lösungsmittels erhält man einen Feststoff.
Rohausbeute: 126 g (86 % d.Th), Fp. 125°C. DAs Produkt ist nach NMR für die Folgeumsetzung genügend rein. Die Verbindung ist aus Cyclohexan umkristallisierbar und hat dann Fp. 160°C.
¹H NMR (CDCl₃, 270 MHz) δ [ppm] : 8,08 (d,1H), 7,85 (d,lH), 3,25 (s,3H), für NCH₃), 3,0 (s,3H)

### Stufe 7: Verseifung zu 2-Methyl-4-(N-methylsulfamoyl)-isophthalsäure

Man suspendiert 127,4 g (0,54 mol) 2,4-Dimethyl-5-cyanosaccharin in einer Mischung von 248,4 g (2,15 mol) 50 proz. KOH und 250 ml Wasser und erhitzt 8 Stunden zum Sieden. Ammoniak entweicht über den Kühler und ist durch feuchtes pH-Papier nachzuweisen.

Zur Aufarbeitung wird mit 500 ml Wasser verdünnt, mit Diethylether extrahiert und die organische Phase verworfen. Die alkalische Wasserphase wird dann mit konz. HCl auf pH 1 gebracht, mit NaCl gesättigt und dreimal mit einer Mischung aus Essigester/THF = 2:1 extrahiert. Nach Trocknen der organischen Phase über Natriumsulfat wird zur Trockne eingeengt. Der Rückstand (132 g, ca. 89 % d.Th.) ist nach IR und ¹H-NMR 2-Methyl-4-(N-methylsulfamoyl)-isophthalsäure. Das Rohprodukt (Fp. 156 bis 158°C braucht für die Umwandlung zum Saccharincarbonsäurechlorid nicht weiter gereinigt zu werden. Der Schmelzpunkt der reinen Verbindung beträgt 180°C.
IR (cm⁻¹): 3306, 3159, 1740, 1714, 1330, 1295, 1266, 1241, 1161

### Stufe 8: 2,4-Dimethyl-saccharin-5-carbonsäurechlorid

132 g (0,484 mol) 2-Methyl-4-(N-methylsulfamoyl)-isophthalsäure werden in 600 ml Toluol suspendiert und mit 0,5 DMF als Katalysator versetzt. Dann läßt man ohne Kühlung (bei 25 bis 50°C) 173 g (1,453 mol) Thionylchlorid aus einem Tropftrichter zulaufen und erhitzt anschließend zum Sieden, bis die Abgasentwicklung abgeklungen ist, was nach etwa vier Stunden der Fall ist.

Das Produktgemisch wird nach dem Abkühlen auf 50°C über Natriumsulfat abgesaugt, um ungelöste Bestandteile zu entfernen. Das Filtrat wird dann im Vakuum eingeengt. Rohausbeute: 115 g (87 % d.Th.). Die Verbindung ist aus Toluol/Cyclohexan = 4:1 umkristallisierbar und hat dann Fp. 172°C. Ausbeute nach Umkristallisation: 92,2 g (78 % d.Th.)
¹H NMR (CDCl₃, 270 MHz) δ [ppm]:8,45 (d,1H), 7,95 (d,1H), 3,25 (s,3H, für NCH₃), 3,0 (d,3H)

In analoger Weise werden erhalten:

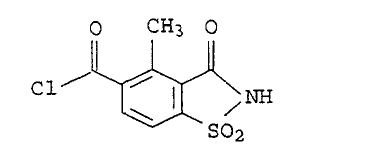

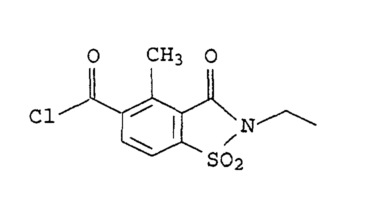

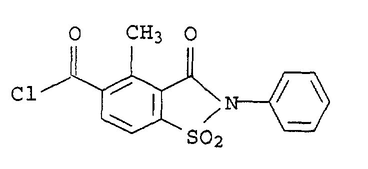

Aus den erfindungsgemäßen Saccharincarbonsäurehalogeniden lassen sich die entsprechenden Saccharincarbonsäuren durch Hydrolyse mit beispielsweise wäßrigem Natriumhydrogencarbonat herstellen oder die Saccharincarbonsäureester durch Umsetzung mit Alkoholaten.

## Patentansprüche

1. Verfahren zur Herstellung von Saccharincarbonsäurehalogeniden der allgemeinen Formel 1, 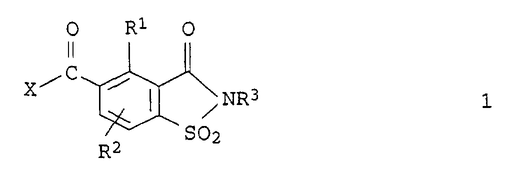 in der die Substituenten die folgende Bedeutung haben:
X Halogen,
R¹, R² Wasserstoff, C₁-C₆-Alkyl, C₃-C₈-Cycloalkyl, C₁-C₄-Alkoxy, ggf. durch C₁-C₄-Alkyl, Fluor oder C₁-C₄-Alkoxy substituiertes Benzyl oder Phenyl,
R³ Wasserstoff, C₁-C₆-Alkyl, C₃-C₈-Cycloalkyl sowie ggf. durch C₁-C₄-Alkyl, Halogen, C₁-C₄-Halogenalkyl oder C₁-C₄-Alkoxy ein- oder mehrfach substituiertes Benzyl oder Phenyl,
dadurch gekennzeichnet, daß man ein Isophthalsäuresulfonamid 2 mit einem anorganischen Säurehalogenid zum Saccharincarbonsäurehalogenid 1 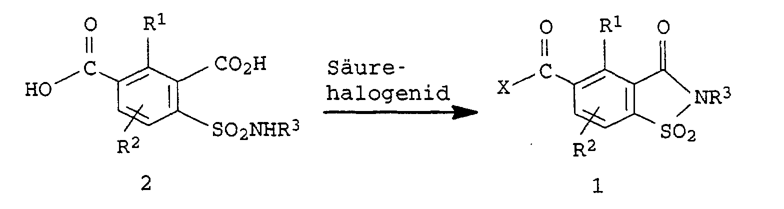 umsetzt.

2. Verfahren nach Anspruch 1, dadurch gekennzeichnet, daß man das Isophthalsäuresulfonamid 2 durch Verseifung eines 5-Cyanosaccharins 3 mit wäßriger Alkalilauge 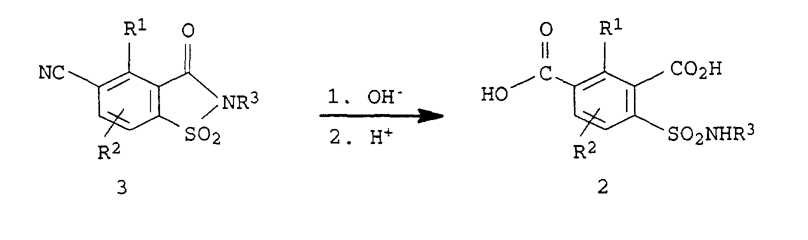 und anschließender Zugabe einer Säure herstellt.

3. Verfahren nach Anspruch 2, dadurch gekennzeichnet, daß man das 5-Cyanosaccharin 3 durch Umsetzung eines Dicyanobenzolsulfonylchlorids 4 mit einem Amin der Formel R³-NH₂ zum Iminosaccharinderivat 5 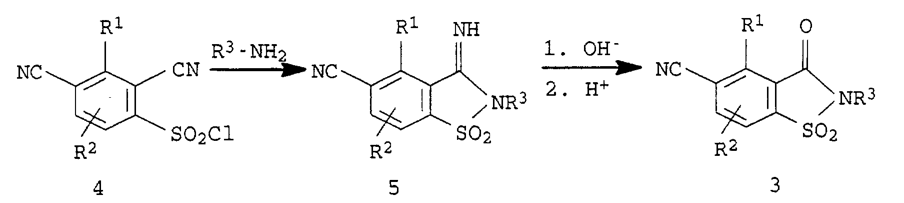 und anschließender saurer Hydrolyse herstellt.

4. Verfahren nach Anspruch 1, dadurch gekennzeichnet, daß man Thionylchlorid, Phosphoroxychlorid, Oxalylchlorid oder Phosgen als anorganisches Säurehalogenid verwendet.

5. Verfahren nach Anspruch 1, dadurch gekennzeichnet, daß man die Umsetzung in einem organischen Lösungsmittel durchführt.

6. Verfahren nach Anspruch 5, dadurch gekennzeichnet, daß man die Umsetzung in Toluol, Chlorbenzol, Xylolen oder 1,2-Dichlorethan durchführt.

7. Verfahren nach Anspruch 1, dadurch gekennzeichnet, daß man die Umsetzung bei einer Temperatur von 25 bis 130°C durchführt.

8. Verfahren nach Anspruch 2, dadurch gekennzeichnet, daß man Natriumhydroxid oder Kaliumhydroxid als Alkalilauge verwendet.

9. Verfahren nach Anspruch 3, dadurch gekennzeichnet, daß man als Amin Ammoniak, C₁-C₆-Alkylamin, C₃-C₈-Cycloalkylamin, Anilin oder Benzylamin verwendet, das ggf. im Phenylring durch C₁-C₄-Alkyl, Halogen, C₁-C₄-Halogenalkyl oder C₁-C₄-Alkoxy substituiert ist.

10. Verfahren nach Anspruch 9, dadurch gekennzeichnet, daß man als Amin Methylamin, Ethylamin oder Anilin verwendet.

## Claims

1. A process for preparing saccharincarbonyl halides of the formula 1 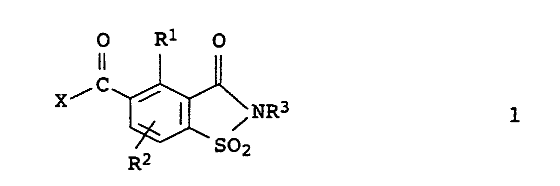 where:
X is halogen,
R¹, R² are each hydrogen, C₁-C₆-alkyl, C₃-C₈-cycloalkyl, C₁-C₄-alkoxy, optionally C₁-C₄-alkyl-, fluorine- or C₁-C₄-alkoxy-substituted benzyl or phenyl,
R³ is hydrogen, C₁-C₆-alkyl, C₃-C₈-cycloalkyl or phenyl or benzyl which is optionally mono- or polysubstituted by C₁-C₄-alkyl, halogen, C₁-C₄-haloalkyl or C₁-C₄-alkoxy,
which comprises reacting an isophthalylsulfonamide 2 with an inorganic acid halide to give the saccharincarbonyl halide 1

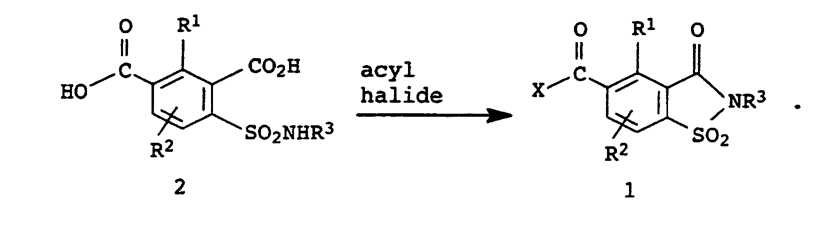

2. A process as claimed in claim 1, wherein the isophthalylsulfonamide 2 is prepared by hydrolysis of a 5-cyanosaccharin 3 with aqueous alkali metal hydroxide solution 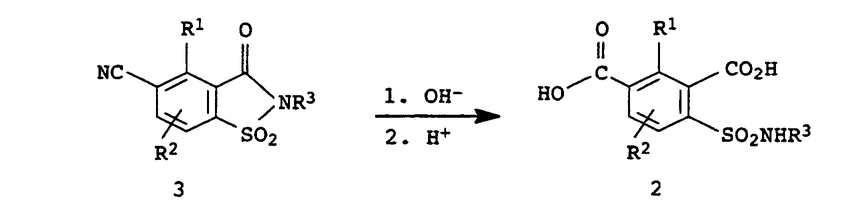 and subsequent addition of an acid.

3. A process as claimed in claim 2, wherein the 5-cyanosaccharin 3 is prepared by reaction of a dicyanobenzenesulfonyl chloride 4 with an amine of the formula R³-NH₂ to give the iminosaccharin derivative 5 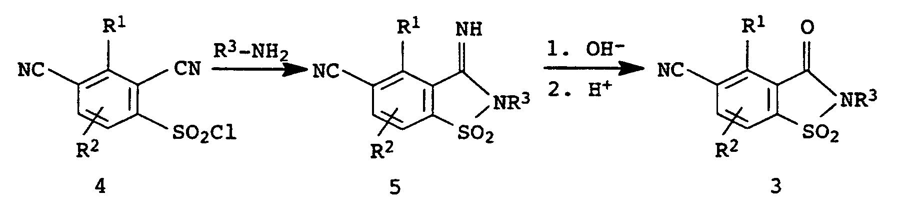 and subsequent acid hydrolysis.

4. A process as claimed in claim 1, wherein the inorganic acid halide used is thionyl chloride, phosphorus oxychloride, oxalyl chloride or phosgene.

5. A process as claimed in claim 1, wherein the reaction is carried out in an organic solvent.

6. A process as claimed in claim 5, wherein the reaction is carried out in toluene, chlorobenzene, xylenes or 1,2-dichloroethane.

7. A process as claimed in claim 1, wherein the reaction is carried out at 25 - 130°C.

8. A process as claimed in claim 2, wherein the alkali metal hydroxide solution used is sodium hydroxide or potassium hydroxide.

9. A process as claimed in claim 3, wherein the amine used is ammonia, C₁-C₆-alkylamine, C₃-C₈-cycloalkylamine, aniline or benzylamine which is optionally substituted in the phenyl ring by C₁-C₄-alkyl, halogen, C₁-C₄-haloalkyl or C₁-C₄-alkoxy.

10. A process as claimed in claim 9, wherein the amine used is methylamine, ethylamine or aniline.

## Revendications

1. Procédé pour la préparation d'halogénures d'acide saccharinecarboxylique de formule générale 1, 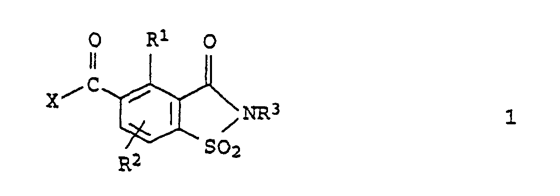 dans laquelle les substituants ont la signification suivante:
X halogéno,
R¹, R², hydrogène, alkyle en C₁-C₆, cycloalkyle en C₃-C₈, alcoxy en C₁-C₄, benzyle ou phényle éventuellement substitué par des groupes alkyle en C₁-C₄, fluor ou alcoxy en C₁-C₄,
R³ hydrogène, alkyle en C₁-C₆, cycloalkyle en C₃-C₈, ainsi que benzyle ou phényle éventuellement mono- ou plurisubstitué par des groupes alkyle en C₁-C₄, halogéno, halogénoalkyle en C₁-C₄ ou alcoxy en C₁-C₄,
caractérisé par le fait qu'on fait réagir un sulfonamide d'acide isophtalique 2 avec un halogénure d'acide minéral pour former l'halogénure d'acide saccharinecarboxylique 1

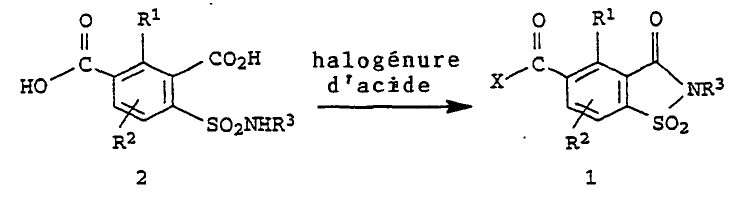

2. Procédé selon la revendication 1, caractérisé par le fait qu'on prépare le sulfonamide d'acide isophtalique 2 par saponification d'une 5-cyanosaccharine 3 avec une lessive alcaline aqueuse 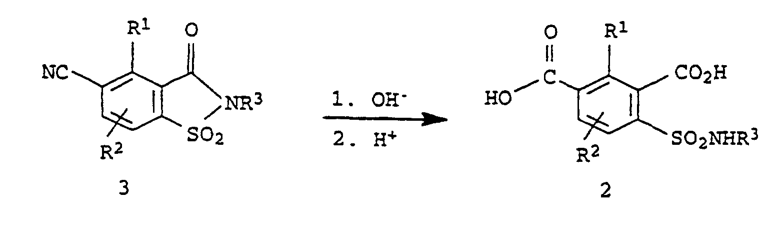 et addition subséquente d'un acide.

3. Procédé selon la revendication 2, caractérisé par le fait qu'on prépare la 5-cyanosaccharine 3 par réaction d'un chlorure de dicyanobenzènesulfonyle 4 avec une amine de formule R³-NH₂ pour former le dérivé d'iminosaccharine 5 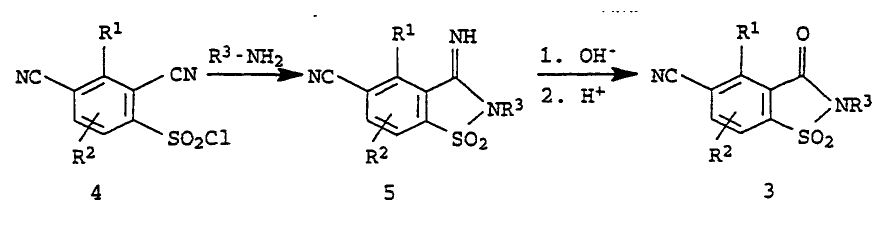 et hydrolyse acide subséquente.

4. Procédé selon la revendication 1, caractérisé par le fait qu'on utilise du chlorure de thionyle, de l'oxychlorure de phosphore, du chlorure d'oxalyle ou du phosgène comme halogénure d'acide minéral.

5. Procédé selon la revendication 1, caractérisé par le fait qu'on effectue la réaction dans un solvant organique.

6. Procédé selon la revendication 5, caractérisé par le fait qu'on effectue la réaction dans du toluène, du chlorobenzène, des xylènes ou du 1,2-dichloroéthane.

7. Procédé selon la revendication 1, caractérisé par le fait qu'on effectue la réaction à une température de 25 à 130°C.

8. Procédé selon la revendication 2, caractérisé par le fait qu'on utilise de l'hydroxyde de sodium ou de l'hydroxyde de potassium en tant que lessive alcaline.

9. Procédé selon la revendication 3, caractérisé par le fait qu'on utilise comme amine de l'ammoniac, de l'alkylamine en C₁-C₆, de la cycloalkylamine en C₃-C₈, de l'aniline ou de la benzylamine, qui est éventuellement substituée dans le cycle phényle par des groupes alkyle en C₁-C₄, halogéno, halogénoalkyle en C₁-C₄ ou alcoxy en C₁-C₄.

10. Procédé selon la revendication 9, caractérisé par le fait qu'on utilise comme amine la méthylamine, l'éthylamine ou l'aniline.
